# EUROPEAN PATENT SPECIFICATION

(11) **EP 4 518 922 B1**
(45) Date of publication and mention of the grant of the patent: **03.12.2025**
(21) Application number: 23724636.8
(22) Date of filing: 25.04.2023
(51) Int. Cl.: A61L 29/08, A61L 29/16

(54) **HYDROPHILIC URINARY CATHETER PRODUCTS WITH MICROCAPSULES OF ANTI-BACTERIAL AGENTS**
HYDROPHILE HARNKATHETERPRODUKTE MIT MIKROKAPSELN AUS ANTIBAKTERIELLEN MITTELN
PRODUITS DE CATHÉTER URINAIRE HYDROPHILE AVEC DES MICROCAPSULES D'AGENTS ANTIBACTÉRIENS

(30) Priority: 06.05.2022 US 202263339172 P
(43) Date of publication of application: 12.03.2025
(73) Proprietor: Hollister Incorporated, Libertyville, IL 60048-3781 (US)
(72) Inventor: PANESAR, Satwinder, S., Libertyville, IL 60048 (US)
(74) Representative: FRKelly
(86) International application number: PCT/US2023/066163
(87) International publication number: WO 2023/215683

(56) References cited:
- WO-A1-2019/222644
- US-A1- 2010 086 580

## Description

The present application claims the benefit of and priority to U.S. Provisional Application No. 63/339,172, filed May 6, 2022.

### FIELD OF THE INVENTION

This disclosure generally relates to a urinary catheter product including anti-microbial microcapsules, wherein the microcapsules are incorporated in a hydrophilic coating or in a hydration solution of the product. More particularly, this disclosure relates to urinary catheter products with microcapsules that release an anti-microbial agent when exposed to irradiation.

### BACKGROUND

Intermittent catheterization is a good option for many users who suffer from various abnormalities of the urinary system. With the advent of intermittent urinary catheters, individuals with problems associated with the urinary system can conveniently self-catheterize to drain the individual's bladder. Individuals who suffer from urinary incontinence will self-catheterize several times a day.

Although catheters are typically prepared in sterile environments and are provided with safe handling instructions, catheter users are at risk of contracting a urinary tract infection due to several different factors. For example, the catheters may become contaminated during use and introduce bacteria into the urethra. The catheter also may carry bacteria along the urinary tract.

US 2010/086580 A discloses a urinary catheter comprising a substrate material, a hydrophilic surface coating arranged on the surface of said substrate material, and an antibacterial layer comprising oligodynamic metal arranged between said substrate material and said hydrophilic surface coating, wherein said hydrophilic surface coating has a thickness large enough to provide a controlled release of oligodynamic metal ions through the hydrophilic surface coating.

Therefore, there is a need for methods and devices that allow catheter users to prevent urinary tract infections.

### SUMMARY OF INVENTION

In one aspect, a urinary catheter product is provided. The urinary catheter product includes a package containing a urinary catheter and a hydration medium. The urinary catheter includes a catheter tube having a proximal insertion end and a distal drainage end. The urinary catheter further includes a hydrophilic polymer coating on an outer surface of the catheter tube. The urinary catheter product also includes a plurality of microcapsules inside the package, wherein the microcapsules include a polysaccharide encapsulating an anti-microbial agent. The microcapsules are configured to release the anti-microbial agent upon irradiation.

In another aspect, a urinary catheter product is provided. The urinary catheter product includes a package containing a urinary catheter and a hydration medium. The urinary catheter includes a catheter tube having a proximal insertion end and a distal drainage end. The urinary catheter further includes a hydrophilic polymer coating on an outer surface of the catheter tube. The urinary catheter product also includes a plurality of microcapsules inside the package, wherein the microcapsules include an encapsulate encapsulating an anti-microbial agent. The microcapsules are configured to release the anti-microbial agent upon exposure to a pH range.

In yet another aspect, a method for making an anti-microbial catheter is provided. The method includes dissolving a polysaccharide into an aqueous solution and adding an anti-microbial agent to the aqueous solution. The method also includes drying the aqueous solution to create a plurality of microcapsules, wherein the microcapsules include the polysaccharide encapsulating the antimicrobial agent. Furthermore, the method includes adding the plurality of microcapsules to a package configured to store the catheter and the hydration medium and releasing the anti-microbial agent.

### BRIEF DESCRIPTION OF DRAWINGS

Fig. 1 is a plan view of an embodiment of the urinary catheter product;
Fig. 2 is an enlarged cross-sectional schematic view of an embodiment of the microcapsule;
Fig. 3 is a side view of a portion of the catheter and an enlarged inset of the catheter outer surface of the urinary catheter product of Fig. 1;
Fig. 4 is a plan view of another embodiment of the urinary catheter product;
Fig. 5 is a cross-sectional view of the urinary catheter product of Fig. 4;
Fig. 6 is a flowchart setting forth the steps in the method of creating an anti-microbial catheter according to an aspect of the present disclosure.

### DETAILED DESCRIPTION

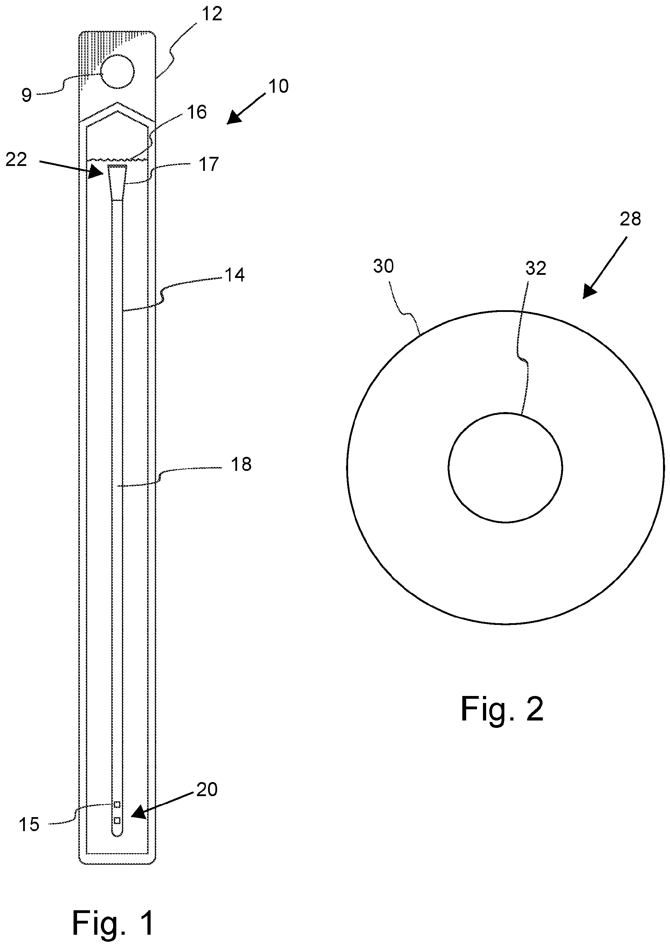

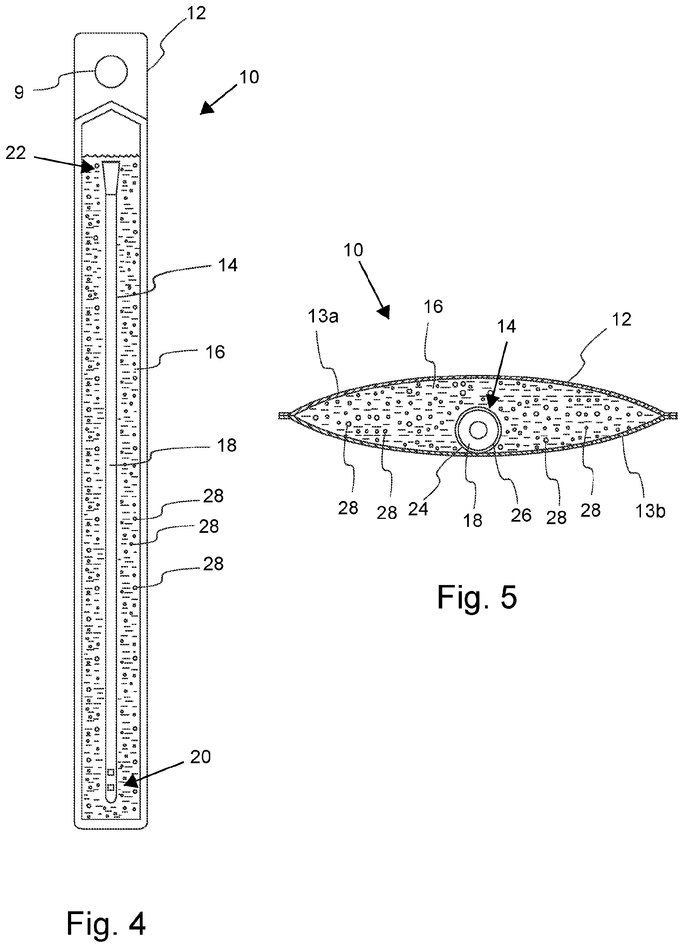

Figs. 1 and 4 show a urinary catheter product 10. The catheter product 10 includes a package 12 containing a urinary catheter 14 and a hydration medium 16. The package includes a cavity configured to receive the urinary catheter 14 and the hydration medium 16. In one embodiment the package 12 may be made of first and second opposing sheets 13a and 13b (shown in Fig. 5). The sheets may be sealed along the edges, to define the cavity. In some embodiments, the package may have a gripping member 9 to assist the user in holding the package 12. In some embodiments, the package 12 may have a tear strip (not shown) to assist the user in opening the package 12. The package may have a generally rectangular shape. Other packages known in the art configured to contain a catheter and hydration medium may be used without departing from the scope of the disclosure.

Fig. 1 further shows urinary catheter 14 inside the package 12. Urinary catheter 14 includes a catheter tube 18 having a proximal insertion end 20 and a distal drainage end 22. A lumen extends from the proximal insertion end 20 to the distal drainage end 22. The proximal insertion end 20 includes one or more openings 15 for receiving urine in communication with the lumen. In one embodiment, the one or more openings 15 for receiving urine may be an eyelet. In another embodiment, the one or more openings 15 for receiving urine may be multiple eyelets. The one or more openings 15 for receiving urine may vary without departing from the scope of the disclosure.

Furthermore, the distal drainage end 22 of the catheter tube 18 includes a drainage opening for draining urine from the lumen. In one embodiment, the distal drainage end 22 includes a drainage member 17. The drainage member 17 may be, for example, but not limited to, a funnel or connector for connection to a drainage bag (not shown). Other drainage members known in the art may be used without departing from the scope of the disclosure.

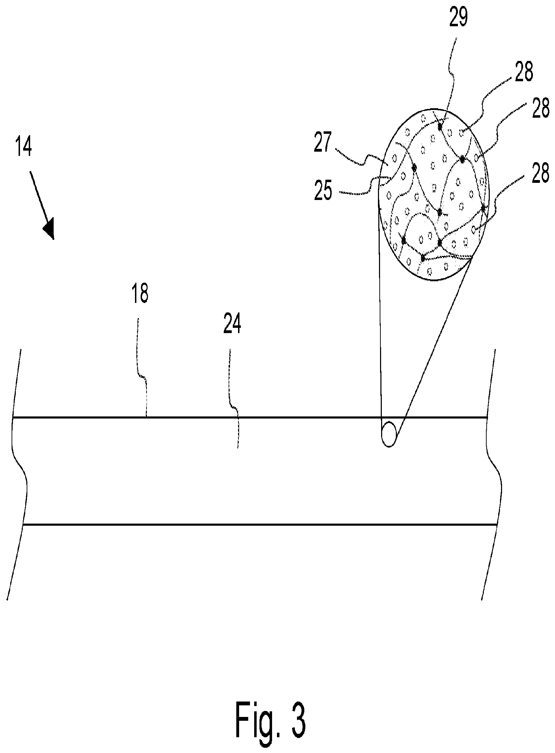

The urinary catheter tube 18 includes a hydrophilic polymer coating 24 (Figs. 3 and 5) on an outer surface 26 of the catheter tube 18. The hydrophilic polymer coating 24 is configured to absorb the hydration medium 16 to lubricate the catheter to aid in insertion into a urethra. Before packaging, the catheter tube 18 may be coated with a hydrophilic polymer solution and then cured to form the hydrophilic coating 24.

The hydrophilic coating solution may include a suitable solvent (such as water and/or ethanol), hydrophilic polymer(s) and optional additives. The resulting hydrophilic coating may include any suitable hydrophilic polymer, such as polyvinylpyrrolidone, polyethylene oxide or polyvinyl alcohol. Examples of hydrophilic polymer solutions used to create a hydrophilic polymer coating and hydrophilic coatings are disclosed in WO2016/168461, filed April 14, 2016, to Hollister. Such hydrophilic coating solutions may include a solvent, hydrophilic polymer(s), other monomers/oligomers and optional additives. In one alternative, the hydrophilic coatings may include a hydrophilic polymer and polyethylene glycol diacrylate having a number average molecular weight of less than about 1000. In one embodiment the number average molecular weight may be less than about 600. In one alternative, the hydrophilic coating comprises about 80 wt% to about 99.5 wt% hydrophilic polymer and about 0.5 wt% to about 20 wt% polyethylene glycol diacrylate. Other hydrophilic polymer solutions and coatings may be used without departing from the scope of the disclosure. During the curing process, the hydrophilic polymer solution crosslinks to create a hydrophilic polymer coating 24 containing a polymer matrix 25 with interstices 27 (shown in Fig. 3).

A plurality of microcapsules 28 is located in the package 12 of the urinary catheter product 10. In one embodiment, the microcapsules 28 are incorporated in the hydrophilic polymer coating 24. In another embodiment the microcapsules 28 are incorporated in the hydration medium 16. In yet, another embodiment, the microcapsules are incorporated into both the hydrophilic polymer coating 24 and the hydration medium 16. Additionally, the hydration medium may be a carrier that carries the microcapsules into the matrix of the hydrophilic polymer coating 24.

Fig. 2 is a schematic illustration of a microcapsule 28. The microcapsule 28 includes an encapsulate 30 encapsulating an anti-microbial agent 32. Preferably, the anti-microbial agent 32 aids in the reduction of urinary tract infections. Upon exposure to a selected condition, the encapsulate 30 breaks down to release the anti-microbial agent 32. In one alternative, the encapsulate 30 breaks down when exposed to irradiation at a dose between about 25kGy and about 65kGy. For example, the encapsulate/microcapsule may liquefy when exposed at a selected radiation dose, thereby releasing the anti-microbial agent. The selected dose may be the same dose used for curing the hydrophilic coating or radiation sterilization of the catheter product. Furthermore, the radiation may be E-beam or gamma. In another alternative, the encapsulate/microcapsule breaks down when exposed to a selected pH. For example, the encapsulate/microcapsule may break down when it is exposed to a pH of about 3.5 - 7.

In one embodiment, the anti-microbial agent 32 is a biocide. Suitable biocides may be silver, antibiotics with biocidal properties such as vancomycin, sparfloxacin, minocycline, and rifampin among others, and poly(catechins)-antibiotic conjugates such as polycatechin with trimethoprim (TMP) and/or polycatechin with TMP and sulfamethoxazole (SMZ). In another embodiment, the anti-microbial agent 32 may have steric repulsion, electrostatic repulsion, and/or low surface energy properties. In yet another embodiment, the anti-microbial agent 32 may be an antifouling material. Suitable antifouling materials include, but are not limited to, hydrophilic materials such as SAM-OEG, PEG, POEGMA; polyzwitterions such as polyMPC, polyCBMA, polySBMA; phosphorylcholines; sulfobetaines; and carboxybetaines. In yet another embodiment, the anti-microbial agent 32 may be any other suitable anti-microbial agent, such as chlorhexidine, amphotercin, bacteriophages, nitrofural, triclosan, anti-microbial peptides, and/or allantoin. In another embodiment, the anti-microbial agent 32 may be an enzyme. Suitable enzymes include hydrolytic enzymes such as proteolytic enzymes (e.g. subtilisin and lysostaphin), polysaccharide hydrolyzing enzymes (e.g. alpha amylase, dispersin B, chitinases, beta-glucanases, lysozyme, and alginate lyase), DNases, and bacteriophage lysins. In yet another embodiment, the anti-microbial agent 32 may be nitric oxide. In yet another embodiment, the anti-microbial agent 32 may be liposomes such as spherical lipid vesicles that consist of one or more phospholipid bilayers. Other anti-microbial agents known in the art may be used without departing from the scope of the disclosure.

The encapsulate 30 may be made of a variety of materials, such as starch, starch derivatives, proteins, gelatins, gums, lipids, natural and synthetic polymers, or any combination of such encapsulates. Examples of natural polymers include, but are not limited to, protein-based polymers and polysaccharide-based polymers. Suitable protein-based polymers include, but are not limited to, albumin and gelatin. Suitable polysaccharide-based polymers include, but are not limited to, agarose, alginate, hyaluronic acid, dextran, gellan gum, chitosan, among others. Examples of synthetic polymers include, but are not limited to, poly(E-caprolactone), poly(lactic-co-glycolic acid), and polyethylene glycol. Other encapsulates known in the art may be used without departing from the scope of the disclosure.

In one alternative, the microcapsules 28 can be made by dissolving the polysaccharide in a solute to form a homogenous solution. In one alternative, the polysaccharide in an amount greater than about 0.5% w/w or about 1.0% w/w, for example, between about 0.5% w/w and about 3% w/w may be dissolved in water. The solution may be heated to assist in dissolving the polysaccharide in the water. For example, gellan gum in an amount greater than about 0.5% w/w or about 1.0% w/w, for example, between about 0.5% w/w and about 3% w/w is dissolved in water at a temperature that is greater than 75° C. Once dissolved, a selected amount/concentration of the anti-microbial agent 32 may be added to the polysaccharide aqueous solution. In one alternative, the selected amount of anti-microbial added to the solution may be between about 1% w/w and about 50% w/w. For example, between about 5% w/w and about 25% w/w of anti-microbial may be added to a gellan gum solution. After the anti-microbial agent 32 has been added, the solution is dried to encapsulate the anti-microbial agent 32 in the polysaccharide. For example, the solution may be cooled to a selected temperature to solidify the polysaccharide and to form a microcapsule encapsulating the anti-microbial therein. When gellan gum is the polysaccharide, the solution may be cooled to below 37° C, thereby commencing solidification of the gellan gum. To assist with drying the polysaccharide encapsulate at temperatures, spray-drying and spin-drying methods may be employed. Other methods of encapsulation may also be used, such as freeze drying, vacuum drying, extrusion, spray cooling, fluidized bed coating, centrifugal extrusion, co-crystallization, molecular inclusion, pan coating, air suspension coating, coacervation, solvent evaporation, and polymerization among other methods known in the art.

The microcapsules 28 may be 10µm - 5mm. Preferably, the microcapsules 28 are 50µm - 1mm. In one embodiment, the size of the microcapsule 28 may be varied based on the pressure of spray-drying the encapsulate 30. In another embodiment the size of the microcapsule 28 may be varied based on the speed of spin-drying the encapsulate 30. In a further embodiment, the size of the microcapsule 28 may be reduced by grinding. The smaller size of the microcapsule 28 aids with incorporation into the hydrophilic polymer coating 24 or the hydration medium 16. The shape of the microcapsule may be spherical or elliptical.

In one embodiment, the microcapsules 28 may be incorporated into the hydrophilic polymer coating 24 on the outer surface 26 of the catheter tube 18. The microcapsules 28 may be incorporated into the coating 24 by adding them to a hydrophilic polymer coating solution. The microcapsules may be added to the hydrophilic polymer coating solution at a concentration between 0.01% w/w to 5.0% w/w. The coating solution may include water and/or ethanol along with the hydrophilic polymer and optional additives. When gellan gum is used, at ambient temperature, the gellan gum microcapsules 28 are insoluble in ethanol and mostly insoluble in water and will suspend in the polymeric coating solution primarily due to their outer surface becoming softened and sticky by aqueous fraction.

After the microcapsules 28 have been suspended in the hydrophilic polymer coating solution, the outer surface 26 of the catheter tube 18 may be coated. The catheter tube may be coated by dip-coating or spray-coating. Other methods of coating a catheter known in the art may be used without departing from the scope of the disclosure.

Once the catheter tube 18 has been coated, the catheter 14 may be cured and dried. In an embodiment, the catheter 14 is cured using UV irradiation and dried at 70° C. Other methods of curing and drying may be used without departing from the scope of the disclosure.

Fig. 3 shows a portion of the catheter tube 18 with an enlarged inset showing the hydrophilic polymer coating 24 with microcapsules 28 incorporated into the coating 24. During curing, the hydrophilic polymer solution crosslinks 29 to create a hydrophilic polymer coating 24 with a polymer matrix 25 and interstices 27. Without being chemically bonded to the coating, the microcapsules 28 may be located in the interstices 27 of the polymer matrix 25.

In another embodiment, the microcapsules 28 may be incorporated into the hydration medium 16. Figs. 4 and 5 show a urinary catheter product 10 including a package 12 containing a urinary catheter 14 and a hydration medium 16. The hydration medium may be water or an aqueous solution. Other hydration mediums known in the art may be used. In some embodiments the hydration medium 16 includes a liquid. In other embodiments, the hydration medium 16 may include a sticky xanthan gum matrix. Microcapsules 28 may be incorporated into the hydration medium 16 by suspending them in the medium 16. Preferably, smaller microcapsules 28 are used to aid with suspension of the microcapsules 28 in the hydration medium 16. Depending on the antimicrobial agent, the microcapsule 28 may be any suitable size. In one alternative, the microcapsules 28 may be less than 500 µm. Similarly, the concentration of microcapsules may vary depending on the antimicrobial agent. In one alternative, the microcapsules 28 may be in a concentration range of 0.01% w/w to 10% w/w in the hydration medium 16.

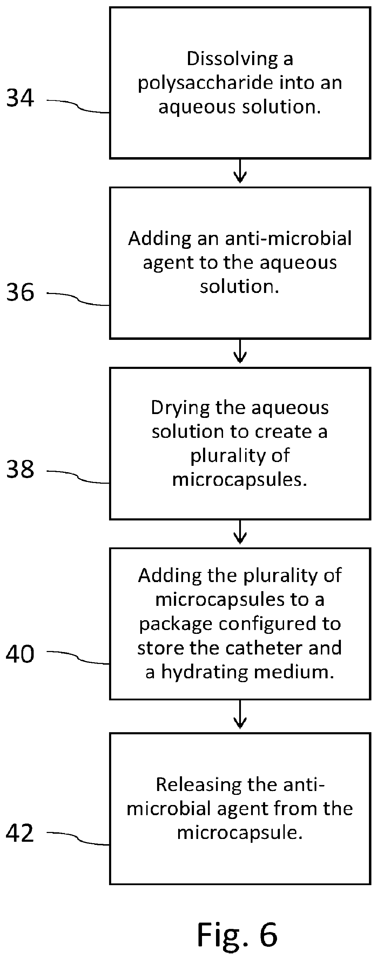

A method for making an anti-microbial catheter 14 is further shown in Fig. 6 in steps 34-42. At step 34, a polysaccharide is dissolved into an aqueous solution. In one embodiment, the polysaccharide may be gellan gum, which readily dissolves into an aqueous solution above 75° C. Other polysaccharides may be used without departing from the scope of the disclosure.

After the polysaccharide is dissolved, at step 36 an anti-microbial agent 32 may be added to the aqueous solution. The anti-microbial agent 32 may be any of the anti-microbial agents described herein.

At step 38, the aqueous solution with the anti-microbial agent 32 is dried to create a plurality of microcapsules 28. The microcapsules 28 include the polysaccharide encapsulating the anti-microbial agent 32. In one embodiment, the solution may be dried by spray-drying or spin-drying. The size of the microcapsules may be varied by adjusting the pressure of spray-drying or the speed of spin-drying. Other drying methods may be used, such as, but not limited to, freeze drying, vacuum drying, extrusion, spray cooling, fluidized bed coating, centrifugal extrusion, co-crystallization, molecular inclusion, pan coating, air suspension coating, coacervation, solvent evaporation, and polymerization among other methods known in the art.

After the microcapsules 28 are created, at step 40 the microcapsules 28 are added to the package 12 configured to store the catheter 14 and hydration medium 16. In one embodiment, the microcapsules 28 are added to the package 12 by being incorporated into the hydrophilic polymer coating 24 on the outer surface 26 of the catheter tube 18. In another embodiment, the microcapsules 28 are added to the package 12 by being incorporated into the hydration medium 16.

After the microcapsules 28 have been added to the package 12, the catheter 14 and hydration medium 16 are sealed in the package 12 and at step 42 the anti-microbial agent 32 may be released from the microcapsule 28.

In one embodiment, the anti-microbial agent 32 may be released upon sterilization of the catheter product 10 by irradiation. The catheter may be sterilized using electron-beam irradiation. Upon electron-beam irradiation, the polysaccharide encapsulate 30, depending on its concentration, will soften or convert to liquid, releasing the anti-microbial agent 32. In the instance where the microcapsules 28 are incorporated into the hydrophilic polymer coating 24, the anti-microbial agent 32 will be released directly onto the outer surface 26 of the catheter tube 18. In the instance where the microcapsules 28 are incorporated into the hydration medium 16, the anti-microbial agent 32 will be released into the hydration medium 16 and then absorbed by the outer surface of the hydrophilic polymer coating 24.

In another embodiment, the anti-microbial agent 32 may be released when the microcapsule is exposed to an environment with a pH within a range of 3.5 - 7. Preferably, the anti-microbial agent 32 is released when the microcapsule is exposed to an environment with a pH of 3.9 - 5. More preferably, the anti-microbial agent 32 is released when the microcapsule is exposed to an environment with a pH of 3.9 - 4.5. The hydration liquid 16 may have a pH configured to release the anti-microbial agent 32. In some instances, the pH of the hydration medium may change upon irradiation, resulting in release of the anti-microbial agent 32.

In yet another embodiment, the anti-microbial agent 32 may be released using temperature or biodegradation with time.

## Claims

1. A urinary catheter product (10) comprising:
a package (12) containing a urinary catheter (14) and a hydration medium (16);
the urinary catheter comprising
a catheter tube (18) having a proximal insertion end (20) and a distal drainage end (22);
a hydrophilic polymer coating (24) on an outer surface (26) of the catheter tube; and
a plurality of microcapsules (28) inside the package, wherein the microcapsules comprise a polysaccharide encapsulating an anti-microbial agent and the microcapsules are configured to release the anti-microbial agent upon irradiation.

2. The urinary catheter product of claim 1, wherein the plurality of microcapsules is incorporated into the hydrophilic polymer coating.

3. The urinary catheter product of any one of claims 1-2, wherein the microcapsules are located within a matrix of the hydrophilic polymer coating.

4. The urinary catheter product of claim 1, wherein the plurality of microcapsules is incorporated into the hydration medium.

5. The urinary catheter product of any one of claims 1-4, wherein the polysaccharide encapsulate comprises gellan gum.

6. The urinary catheter product of any one of claims 1-5, wherein the anti-microbial agent is released upon the microcapsules being exposed to electron-beam irradiation.

7. A urinary catheter product (10) comprising:
a package (12) containing a urinary catheter (14) and a hydration medium (16);
the urinary catheter comprising
a catheter tube (18) having a proximal insertion end (20) and a distal drainage end (22);
a hydrophilic polymer coating (24) on an outer surface (26) of the catheter tube; and
a plurality of microcapsules (28) inside the package, wherein the microcapsules comprise an encapsulate encapsulating an anti-microbial agent and the microcapsules are configured to release the anti-microbial agent upon exposure to a pH range.

8. The urinary catheter product of claim 7, wherein the encapsulate comprises a polysaccharide.

9. The urinary catheter product of any one of claims 7-8, wherein the encapsulate is gellan gum.

10. The urinary catheter product of any one of claims 7-9, wherein the plurality of microcapsules is incorporated into the hydrophilic polymer coating.

11. The urinary catheter product of any one of claims 7-9, wherein the plurality of microcapsules is incorporated into the hydration medium.

12. The urinary catheter product of any one of claims 7-11, wherein the anti-microbial agent is released upon the microcapsules being exposed to a pH of 3.5 to 7.

13. A method for making an anti-microbial catheter (14) comprising:
dissolving a polysaccharide into an aqueous solution;
adding an anti-microbial agent to the aqueous solution;
drying the aqueous solution to create a plurality of microcapsules (28),
wherein the microcapsules comprise the polysaccharide encapsulating the anti-microbial agent;
adding the plurality of microcapsules to a package (12) configured to store the catheter and a hydration medium (16); and
releasing the anti-microbial agent from the microcapsule.

14. The method of claim 13, wherein the microcapsules are incorporated in a hydrophilic polymer coating (24) on an outer surface of the catheter.

15. The method of claim 13, wherein the microcapsules are incorporated in the hydration medium.

16. The method of any one of claims 13-15, wherein the anti-microbial agent is released upon irradiation.

17. The method of any one of claims 13-15, wherein the anti-microbial agent is released upon the microcapsules being exposed to a pH of 3.5 to 7.

## Patentansprüche

1. Harnkatheterprodukt (10), umfassend:
eine Verpackung (12), die einen Harnkatheter (14) und ein Hydrationsmedium (16) enthält;
wobei der Harnkatheter Folgendes umfasst:
einen Katheterschlauch (18) mit einem proximalen Einführungsende (20) und einem distalen Ablaufende (22);
eine hydrophile Polymerbeschichtung (24) auf einer Außenfläche (26) des Katheterschlauchs; und
eine Vielzahl von Mikrokapseln (28) im Inneren der Verpackung, wobei die Mikrokapseln ein Polysaccharid umfassen, das ein antimikrobielles Mittel einkapselt, und die Mikrokapseln dazu konfiguriert sind, das antimikrobielle Mittel bei Bestrahlung freizusetzen.

2. Harnkatheterprodukt nach Anspruch 1, wobei die Vielzahl von Mikrokapseln in die hydrophile Polymerbeschichtung eingearbeitet ist.

3. Harnkatheterprodukt nach einem der Ansprüche 1-2, wobei die Mikrokapseln innerhalb einer Matrix der hydrophilen Polymerbeschichtung angeordnet sind.

4. Harnkatheterprodukt nach Anspruch 1, wobei die Vielzahl von Mikrokapseln in das Hydrationsmedium eingearbeitet ist.

5. Harnkatheterprodukt nach einem der Ansprüche 1-4, wobei die Polysaccharidverkapselung Gellangummi umfasst.

6. Harnkatheterprodukt nach einem der Ansprüche 1-5, wobei das antimikrobielle Mittel freigesetzt wird, wenn die Mikrokapseln einer Elektronenstrahlbestrahlung ausgesetzt werden.

7. Harnkatheterprodukt (10), umfassend:
eine Verpackung (12), die einen Harnkatheter (14) und ein Hydrationsmedium (16) enthält;
wobei der Harnkatheter Folgendes umfasst:
einen Katheterschlauch (18) mit einem proximalen Einführungsende (20) und einem distalen Ablaufende (22);
eine hydrophile Polymerbeschichtung (24) auf einer Außenfläche (26) des Katheterschlauchs; und
eine Vielzahl von Mikrokapseln (28) im Inneren der Verpackung, wobei die Mikrokapseln eine Verkapselung umfassen, die ein antimikrobielles Mittel einkapselt, und die Mikrokapseln dazu konfiguriert sind, das antimikrobielle Mittel bei Aussetzung gegenüber einem pH-Bereich freizusetzen.

8. Harnkatheterprodukt nach Anspruch 7, wobei die Verkapselung ein Polysaccharid umfasst.

9. Harnkatheterprodukt nach einem der Ansprüche 7-8, wobei die Verkapselung Gellangummi ist.

10. Harnkatheterprodukt nach einem der Ansprüche 7-9, wobei die Vielzahl von Mikrokapseln in die hydrophile Polymerbeschichtung eingearbeitet ist.

11. Harnkatheterprodukt nach einem der Ansprüche 7-9, wobei die Vielzahl von Mikrokapseln in das Hydrationsmedium eingearbeitet ist.

12. Harnkatheterprodukt nach einem der Ansprüche 7-11, wobei das antimikrobielle Mittel freigesetzt wird, wenn die Mikrokapseln einem pH von 3,5 bis 7 ausgesetzt werden.

13. Verfahren zum Herstellen eines antimikrobiellen Katheters (14), umfassend:
Auflösen eines Polysaccharids in einer wässrigen Lösung;
Zugeben eines antimikrobiellen Mittels zur wässrigen Lösung;
Trocknen der wässrigen Lösung, um eine Vielzahl von Mikrokapseln (28) zu erzeugen, wobei die Mikrokapseln das Polysaccharid umfassen, das das antimikrobielle Mittel einkapselt;
Hinzufügen der Vielzahl von Mikrokapseln zu einer Verpackung (12), die dazu konfiguriert ist, den Katheter und ein Hydrationsmedium (16) aufzubewahren; und
Freisetzen des antimikrobiellen Mittels aus der Mikrokapsel.

14. Verfahren nach Anspruch 13, wobei die Mikrokapseln in eine hydrophile Polymerbeschichtung (24) auf einer Außenfläche des Katheters eingearbeitet sind.

15. Verfahren nach Anspruch 13, wobei die Mikrokapseln in das Hydrationsmedium eingearbeitet sind.

16. Verfahren nach einem der Ansprüche 13-15, wobei das antimikrobielle Mittel bei Bestrahlung freigesetzt wird.

17. Harnkatheterprodukt nach einem der Ansprüche 13-15, wobei das antimikrobielle Mittel freigesetzt wird, wenn die Mikrokapseln einem pH von 3,5 bis 7 ausgesetzt werden.

## Revendications

1. Produit de cathéter urinaire (10) comprenant :
un emballage (12) contenant un cathéter urinaire (14) et un milieu d'hydratation (16) ;
le cathéter urinaire comprenant
un tube de cathéter (18) ayant une extrémité d'insertion proximale (20) et une extrémité de drainage distale (22) ;
un revêtement polymère hydrophile (24) sur une surface extérieure (26) du tube de cathéter ; et
une pluralité de microcapsules (28) à l'intérieur de l'emballage, dans lequel les microcapsules comprennent un polysaccharide encapsulant un agent antimicrobien et les microcapsules sont configurées pour libérer l'agent antimicrobien lors de l'irradiation.

2. Produit de cathéter urinaire selon la revendication 1, dans lequel la pluralité de microcapsules sont incorporées dans le revêtement polymère hydrophile.

3. Produit de cathéter urinaire selon l'une quelconque des revendications 1 et 2, dans lequel les microcapsules sont situées à l'intérieur d'une matrice du revêtement polymère hydrophile.

4. Produit de cathéter urinaire selon la revendication 1, dans lequel la pluralité de microcapsules sont incorporées dans le milieu d'hydratation.

5. Produit de cathéter urinaire selon l'une quelconque des revendications 1 à 4, dans lequel l'encapsulat de polysaccharide comprend de la gomme gellane.

6. Produit de cathéter urinaire selon l'une quelconque des revendications 1 à 5, dans lequel l'agent antimicrobien est libéré lorsque les microcapsules sont exposées à une irradiation par faisceau d'électrons.

7. Produit de cathéter urinaire (10) comprenant :
un emballage (12) contenant un cathéter urinaire (14) et un milieu d'hydratation (16) ;
le cathéter urinaire comprenant
un tube de cathéter (18) ayant une extrémité d'insertion proximale (20) et une extrémité de drainage distale (22) ;
un revêtement polymère hydrophile (24) sur une surface extérieure (26) du tube de cathéter ; et
une pluralité de microcapsules (28) à l'intérieur de l'emballage, dans lequel les microcapsules comprennent un encapsulat encapsulant un agent antimicrobien et les microcapsules sont configurées pour libérer l'agent antimicrobien lors de l'exposition à une plage de pH.

8. Produit de cathéter urinaire selon la revendication 7, dans lequel l'encapsulat comprend un polysaccharide.

9. Produit de cathéter urinaire selon l'une quelconque des revendications 7 et 8, dans lequel l'encapsulat est de la gomme gellane.

10. Produit de cathéter urinaire selon l'une quelconque des revendications 7 à 9, dans lequel la pluralité de microcapsules sont incorporées dans le revêtement polymère hydrophile.

11. Produit de cathéter urinaire selon l'une quelconque des revendications 7 à 9, dans lequel la pluralité de microcapsules sont incorporées dans le milieu d'hydratation.

12. Produit de cathéter urinaire selon l'une quelconque des revendications 7 à 11, dans lequel l'agent antimicrobien est libéré lorsque les microcapsules sont exposées à un pH de 3,5 à 7.

13. Procédé de fabrication d'un cathéter antimicrobien (14) comprenant :
la dissolution d'un polysaccharide dans une solution aqueuse ;
l'ajout d'un agent antimicrobien à la solution aqueuse ;
le séchage de la solution aqueuse pour créer une pluralité de microcapsules (28), dans lequel les microcapsules comprennent le polysaccharide encapsulant l'agent antimicrobien ;
l'ajout de la pluralité de microcapsules à un emballage (12) configuré pour stocker le cathéter et un milieu d'hydratation (16) ; et
la libération de l'agent antimicrobien de la microcapsule.

14. Procédé selon la revendication 13, dans lequel les microcapsules sont incorporées dans un revêtement polymère hydrophile (24) sur une surface extérieure du cathéter.

15. Procédé selon la revendication 13, dans lequel les microcapsules sont incorporées dans le milieu d'hydratation.

16. Procédé selon l'une quelconque des revendications 13 à 15, dans lequel l'agent antimicrobien est libéré lors de l'irradiation.

17. Procédé selon l'une quelconque des revendications 13 à 15, dans lequel l'agent antimicrobien est libéré lorsque les microcapsules sont exposées à un pH de 3,5 à 7.
